# EUROPEAN PATENT SPECIFICATION

(11) **EP 2 377 516 B1**
(45) Date of publication and mention of the grant of the patent: **20.06.2012**
(21) Application number: 10159870.4
(22) Date of filing: 14.04.2010
(51) Int. Cl.: A61K 9/10, A61K 47/36, A61K 31/167

(54) **Acetaminophen composition**
Acetaminophenzusammensetzung
Composition de paracétamol

(43) Date of publication of application: 19.10.2011
(73) Proprietor: B. Braun Melsungen AG, 34212 Melsungen (DE)
(72) Inventor: Béjar, Juan Gil, 08191, Rubí, Barcelona (ES); García, Jesús Iglesias, 08006, Barcelona (ES)
(74) Representative: von Kreisler Selting Werner

(56) References cited:
- WO-A1-00/48637
- WO-A2-02/072080

## Description

The invention relates to a liquid injectable composition comprising acetaminophen, hydroxyethylstarch and at least one osmolality agent. Further, the invention relates to a pharmaceutical composition for the prophylaxis and treatment of pain and fever containing said injectable composition. Additionally, the invention relates to a process for the manufacturing of the composition as well as a container comprising said liquid injectable composition.

It has been known for many years that acetaminophen (paracetamol) in the presence of moisture, and especially in aqueous solution may be hydrolyzed to p-aminophenol which subsequently may itself be converted into quinine-imine. The weight of decomposition of paracetamol is enhanced as the temperature is increased and upon exposure to light.

In addition, the instability of paracetamol in aqueous solution as a function of the solution's pH has been extensively described. Thus, according to the publication Stability of aqueous solution of N-acetyl-p-aminophenol" (Koshy K.T. and Lach J. I. J. Pharm. Sci., 50 (1961), pp. 113-118), paracetamol in aqueous solution is unstable, a fact which primarily correlates with hydrolysis both in acidic and basic environment. This breakdown process is minimal at pH close to 6.

Besides hydrolysis, the paracetamol molecule separately undergoes another kind of decomposition that involves formation of a quinine-imine that may readily polymerize with generation of nitrogen containing polymers.

These polymers and in particular those stemming from N-acetyl-p-benzoquinone-imine (NAPBQI) have been further described as being the toxic metabolite of paracetamol, which is endowed notably with cytotoxic and haemolytic effect.

In the state of the art and in view of the quality control requirements specific to pharmaceutical practice regulations, the stability of paracetamol in aqueous solutions is thus insufficient and does not allow the formulation of liquid pharmaceutical compositions for injection. As a result, the successful preparation of liquid pharmaceutical formulations for parenteral administration, based on paracetamol, has not been achieved.

A number of trials have been undertaken to slow down the decomposition of paracetamol in an aqueous solution. In some works, the addition of EDTA was used to slow down the rate of decomposition of paracetamol.

US-6,028,222 describes a liquid formulation consisting essentially of acetaminophen dispersed in an aqueous medium containing a buffering agent and at least one member of the group consisting of a free radical scavenger and a radical antagonist. To prevent degradation the acetaminophen solutions are deoxygenated by bubbling a water insoluble inert gas such as nitrogen through the aqueous formulation.

WO-A1-2004/071502 discloses an injectable liquid pharmaceutical formulation of paracetamol which contains paracetamol, an aqueous solvent, a buffer with a pKa of between 4.5 and 6.5, an isotonic agent as well as a paracetamol dimer. The paracetamol dimer is used as a stabilizing agent for the aqueous formulation comprising paracetamol.

WO-A2-2009/047634 discloses an aqueous formulation of acetaminophen comprising 200 to 1400 mg of acetaminophen, and 200 to 10000 mg of mannitol. In order to stabilize the formulation against degradation povidon as well as monobasic sodium phosphate is used.

EP-A1-1 992 334 discloses a liquid stable to oxidation formulation comprising paracetamol and an aqueous solvent wherein the formulation is characterized by a pH between 5.0 and 6.0 and an oxygen concentration below 2 ppm. Thus, the formulation described necessarily requires a deoxygenation step in order to stabilize the paracetamol formulation.

EP-A1-1 752 139 discloses a liquid, aqueous formulation comprising paracetamol and an antioxidants selected from the group consisting of ascorbic acid, N-acetyl-L-cystein and SH-group containing stabilizers. Further, it is necessary to keep the oxygen content lower than 1 mg/l.

EP-A1-1 465 663 discloses a ready-to-use pharmaceutical paracetamol injection solution obtainable by mixing paracetamol with water, propylenglycol as the only co-solvent and a citrate buffer by heating said solution from 70°C to 130°C. The paracetamol formulations mandatorily require organic solvents such as propylenglycol. Likewise, EP-A1-1 094 802 discloses a pharmaceutical composition comprising paracetamol as well as ethanol and polyethylene glycol.

EP-A1-1 889 607 discloses an injectable liquid paracetamol formulation. In order to prevent degradation of the paracetamol in the aqueous formulation antioxidants such as sodium formaldehyde sulfoxide is proposed. However, sodium formaldehyde sulfoxylate lead to a release of a certain amount of sodium sulfide which is in fact an organic related metasulfide. Sulfides are known to cause problems and it is well known that many people undergo anaphylactic and/or hypersensitivity reactions due to the presence of sulfide derivatives.

The objective of the present invention is the provision of a pharmaceutical composition comprising acetaminophen which has an improved long term stability in terms of oxidation resistance and hydrolysis stability. Additionally, a process for the manufacturing of a paracetamol formulation is provided which can be prepared easily since no deoxygenation step is required and the acetaminophen can easily be dissolved in water at room temperature.

It has surprisingly found that the above-mentioned problems can be solved by a liquid injectable composition comprising
a) acetaminophen,
b) hydroxyethyl starch and
c) at least one osmolality agent.

Paracetamol (acetaminophen) is a widely used over-the-counter analgesic (pain reliever) and antipyretic (fever reducer). It is commonly used for the relief of fever, headaches and other minor aches and pains and is a major ingredient in numerous cold and flu remedies. In combination with non-stereoidal antiinflammatory drugs (NSAIG) and opioid analgesics, paracetamol is also used in the management of more severe pain (such as cancer or post operative pain).

The systematic IUPAC name of acetaminophen is N-(4-hydroxyphenyl)ethanamide.

The liquid injectable composition according to the present invention preferably comprises acetaminophen in a concentration ranging from 0.05 to 5.0 percent by weight, more preferably from 0.5 to 3.0 percent by weight and especially from 0.8 to 1.8 percent by weight, wherein the amounts referred to are based on the total weight of the composition.

A further essential component of the liquid injectable composition according to the present invention is hydroxyethylstarch. It has surprisingly found that hydroxyethylstarch significantly increases the rate of dissolution of acetaminophen in a solution especially in an aqueous solution. Thus, the presence of hydroxyethylstarch in an acetaminophen formulation increases the solubility of the acetaminophen and, as a consequence, it is possible to dissolve the acetaminophen quickly in an aqueous formulation at lower temperatures, i.e., at a temperature range from 5 to 50°C, preferably 15 to 40°C and more preferably 18 to 30°C. Since it is possible to dissolve the acetaminophen in an aqueous formulation at low temperatures the degree of hydrolyzation and the degree of oxidation products during the manufacturing process can significantly be reduced. Additionally, the presence of hydroxyethylstarch reduces significantly the degradation of an aqueous formulation comprising acetaminophen which is stored at air or in a container which is semipermeable for oxygen. Thus, the liquid injectable compositions according to the present invention do not need to be deoxygenated and, above all, do not need to be stored under an nitrogen atmosphere.

Hydroxyethylstarch (HES) is a well known synthetic colloid. Worldwide, different HES preparations are currently used as colloidal volume replacements, which are mainly distinguished by their molecular weights and additionally by their extent of etherification with hydroxyethyl groups, and by other parameters. The best known representatives of this class of substances are the so-called Hetastarch (HES 450/0.7) and Pentastarch (HES 200/0.5). The latter is the currently most widespread "standard HES". Besides, HES 200/0.62 and HES 70/0.5 play a minor role. The declared information relating to the molecular weight as well as that relating to the other parameters are averaged quantities, where the molecular weight declaration is based on the weight average (Mw) expressed in Daltons (e.g., for HES 200,000) or mostly abbreviated in Kilodaltons (e.g., for HES 200). The extent of etherification with hydroxyethyl groups is characterized by the molar substitution MS (e.g. as 0.5 such as in HES 200/0.5; MS = average molar ratio of hydroxyethyl groups to anhydroglucose units) or by the degree of substitution (DS = ratio of mono- or polyhydroxyethylated glucoses to the total anhydroglucose units). According to their molecular weights, the HES solutions in clinical use are classified into high-molecular weight (450 kD), medium-molecular weight (200-250 kD) and low-molecular weight (70-130 kD) preparations.

The hydroxyethylstarches according to the invention are influenced by the molar substitution MS. The molar substitution MS is defined as the average number of hydroxyethyl groups per anhydroglucose unit (Sommermeyer et al., Krankenhauspharmazie (1987), pp. 271 to 278). The molar substitution can be determined according to Ying-Che Lee et al., Anal. Chem. (1983) 55, 334, and K.L. Hodges et al., Anal. Chem. (1979) 51, 2171. In this method, a known amount of HES is subjected to ether cleavage by adding adipic acid and hydroiodic acid (HI) in xylene. Subsequently, the ethyl iodide released is quantified by gas chromatography using an internal standard (toluene) and external standards (ethyl iodide calibrating solutions). The molar substitution MS influences the effect of the hydroxyethylstarches according to the invention. If the MS is selected too high, this may cause an accumulation effect in the circulation when the hydroxyethylstarches are employed. On the other hand, if the MS is selected too low, this may result in too rapid a degradation of the hydroxyethylstarch in the circulation and thus reduce the desired duration of the plasma half life. A molar substitution MS of 0.3 to 0.7, preferably from 0.35 to 0.5 (0.35 ≤ MS ≤ 0.50), more preferably from 0.39 to smaller than or equal to 0.45 (0.39 ≤ MS ≤ 0.45) and especially an MS of from greater than 0.4 to 0.44 (0.4 < MS ≤ 0.44), has proven advantageous.

The hydroxyethylstarches used according to the invention belong preferably to the higher-molecular weight hydroxyethylstarches and more preferably have an average molecular weight (Mw) ranging from 10,000 to 500,000, even more preferably from 20,000 to 150,000. Due to the preparation conditions, the hydroxyethylstarches are not in the form of a substance with a defined uniform molecular weight but in the form of a mixture of molecules of different sizes which are also differently substituted by hydroxyethyl groups. Therefore, the characterization of such mixtures requires recourse to statistically averaged quantities. Therefore, the weight-average molecular weight (Mw) serves for characterizing the average molecular weight, the general definition of this mean value being stated in Sommermeyer et al., Krankenhauspharmazie (1987), pp. 271 to 278.

The molecular weight determination can be effected by means of GPC-MALLS using the GPC columns TSKgel G 6000 PW, G 5000 PW, G 3000 PW and G 2000 PW (7.5 mm x 30 cm), the MALLS detector (DAWN-EOS; Wyatt Deutschland GmbH, Woldert) and the RI detector (Optilab DSP; Wyatt Deutschland GmbH, Woldert) at a flow rate of 1.0 ml/minute in a 50 mM phosphate buffer, pH 7.0. The evaluation may be performed by means of ASTRA software (Wyatt Deutschland GmbH, Woldert).

Preferred are those hydroxyethylstarches which are obtainable from native or partially hydrolyzed cereal or potato starches. Due to their high content of amylopectin, the use of starches from waxy varieties of the corresponding crops, if they exist (e.g., waxy maize, waxy rice), is particularly advantageous.

The hydroxyethylstarch according to the invention can further be described by the ratio of substitution at C₂ to substitution at C₆ of the anhydroglucose units. This ratio, which is also abbreviated as C₂/C₆ ratio within the scope of this invention, means the ratio of the number of anhydroglucose units substituted in 2 position to the number of anhydroglucose units substituted in 6 position of the hydroxyethylstarch. The C₂/C₆ ratio of an HES can be varied widely by the amount of aqueous sodium hydroxide used in the hydroxyethylation. The higher the amount of NaOH employed, the more highly the hydroxy groups in 6 position in the anhydroglucose of the starch are activated for hydroxyethylation. Therefore, the C₂/C₆ ratio decreases during the hydroxyethylation with increasing NaOH concentration. The determination is effected as stated by Sommermeyer et al., Krankenhauspharmazie (1987), pp. 271 to 278. With increasing preference in the order given, the C₂/C₆ ratios are preferably from 3 to below 8, from 2 to 7, from 3 to 7, from 2.5 to smaller than or equal to 7, from 2.5 to 6, or from 4 to 6.

In principle, all known starches are suitable for the preparation of the hydroxyethylstarches, mainly native or partially hydrolyzed starches, preferably cereal or potato starches, especially those having a high content of amylopectin. In a particular embodiment starches from waxy varieties, especially waxy maize and/or waxy rice, are employed. In a particular embodiment, the preparation of HES is effected by reacting water-suspended cereal and/or potato starch, preferably thin boiling waxy maize starch, with ethylene oxide. Advantageously, the reaction is catalyzed by adding alkalizing agents, preferably alkali metal hydroxides, for example, sodium hydroxide or potassium hydroxide. Preferably, an alkalizing agent, preferably sodium hydroxide, is additionally added to the water-suspended starch. The alkalizing agent is added to the suspended starch preferably in such an amount that the molar ratio of alkalizing agent to starch is greater than 0.2, preferably from 0.25 to 1, especially from 0.3 to 0.8. Through the ratio of ethylene oxide to starch during the hydroxyethylation step, the molar substitution, i.e., the molar ratio of hydroxyethyl groups to anhydroglucose units, can be arbitrarily controlled over the desired MS range. Preferably, the reaction between ethylene oxide and suspended starch is effected in a temperature range of from 30 to 70 °C, preferably from 35 to 45 °C. Usually, any residues of ethylene oxide are removed after the reaction. In a second step following the reaction, an acidic partial hydrolysis of the derivatized starch is effected. "Partial hydrolysis" means the hydrolysis of the alpha-glycosidically interconnected glucose units of the starch. In principle, all acids familiar to the skilled person can be employed for the acidic hydrolysis, but preferred are mineral acids, especially hydrochloric acid. The hydrolysis may also be effected enzymatically using commercially available amylases.

The liquid injectable composition of the present invention comprises the hydroxyethylstarch in an amount preferably ranging from 0.05 to 4 percent by weight, more preferably from 0.08 to 2 percent by weight and especially from 0.1 to 1.5 percent by weight, wherein the amount is based on the total weight of the composition.

A further essential component of the liquid injectable composition according to the present invention is an osmolality agent. The composition according to the present invention comprises at least one osmolality agent. Preferably, the osmolality agent is an isoosmolality agent or an isotonic agent, preferably a nonionic isotonic agent.

In a further preferred embodiment the osmolality agent is an aliphatic polyhydroxy alkanol having 2 to 10 carbon atoms, preferably selected from the group consisting of mannitol, fructose, glucose, gluconolactone, gluconat and mixtures thereof.

Especially preferred is mannitol.

Further preferred osmolality agents are selected from the group consisting of glucose, laevulose, calcium gluconoglucoheptonate, potassium chloride, calcium chloride, sodium chloride and mixtures thereof.

Preferably, the osmolality agent is present in an amount ranging from 0.5 to 10 percent by weight, more preferably 1 to 7 percent by weight and most preferably 1.5 to 5 percent by weight and especially 2 to 4 percent by weight. The amounts referred to are based on the total weight of the composition.

The preferred osmolality of the composition according to the invention is ranging from 250 mOsm/kg to 400 mOsm/kg, more preferably ranging from 290 mOsm/kg to 340 mOsm/kg.

Advantageously, the liquid injectable composition according to the present invention additionally comprises a buffering agent. The buffer which can be used is a buffer compatible with parenteral administration in humans, the pH of which may be adjusted between 4 and 8. Preferred buffers are based on alkali metal or alkaline earth metal acetates or phosphates. A more preferred buffer is sodium acetate/hydrogene phosphate adjusted to the required pH with hydrochloric acid or sodium hydroxide. More preferably the buffering agent is selected from the group consisting of a buffer based on acetate, citrate and phosphate as well as mixtures thereof. Especially preferred is a acetate/citrate buffering agent. In particular, good results have been achieved wherein the buffer is sodium acetate/sodium citrate wherein the required pH is adjusted with acetic acid.

A further preferred buffer system comprises phosphate, preferably disodium hydrogen phosphate and phosphoric acid.

In order to further improve the hydrolytic stability of the acetaminophen present in the liquid injectable composition of the present invention the pH of the composition is desirably adjusted to a pH value ranging from 4 to 8, preferably 4.5 to 6.5 and more preferably from 5.0 to 6.0.

The liquid injectable composition according to the present invention is preferably aqueous. Due to the improved solubility of the acetaminophen in the liquid injectable composition of the present invention organic solvents such as alcohols and/or glycols are not necessary. Therefore, according to a preferred embodiment of the present invention the liquid composition is essentially free of organic solvents, especially essentially free of glycols and/or alcohols. Essentially free within the meaning of the present invention means that the liquid composition comprises less than 10% by weight, preferably less than 5% by weight, more preferably less than 2% by weight and in particular 0% by weight of the respective component wherein the amounts referred to are based on the total weight of the composition.

The composition according to the present invention can further comprise additives or active ingredients which are compatible with parenteral administration in humans.

The liquid injectable composition according to the present invention is suitable to be used as a pharmaceutical composition. Accordingly, a further embodiment of the present invention is a pharmaceutical composition comprising the liquid injectable composition of the present invention.

In particular, the pharmaceutical composition of the present invention is used for the prophylaxis and treatment of pain and/or fever. Preferably, the pharmaceutical composition is administered to the human by injection or infusion.

The composition according to the present invention can easily be prepared. Since the presence of hydroxyethylstarch in an aqueous formulation comprising acetaminophen improves the rate of dissolution of the acetaminophen which at the same time prevents the degradation (hydrolysis as well as oxidative degradation) it is an advantage for the manufacturing process of the composition of the present invention to dissolve the acetaminophen in a solvent in the presence of hydroxyethylstarch. Due to the improved rate of dissolution of the acetaminophen even in aqueous formulations which are essentially free of organic solvents, the manufacturing process of the composition of the present invention can be conducted at lower temperatures than the methods for the preparation of paracetamol formulations disclosed in the prior art.

A further embodiment of the present invention is a process for the manufacturing of a composition of the present invention comprising the step of dissolving acetaminophen in a solvent in the presence of hydroxyethylstarch.

The hydroxyethylstarch to be used in the process of the present invention is already defined above. Preferably, the solvent is an aqueous solvent, preferably water. Since the compositions of the present invention do not necessarily require organic solvents, especially do not require organic alcohols and/or glycols the process of the present invention can be conducted in an aqueous solvent which is preferably essentially free of organic solvents, in particular essentially free of alcohols and/or organic glycols.

Preferably, the acetaminophen is dissolved in the presence of hydroxyethylstarch at a temperature ranging from 5 to 50°C, preferably 15 to 50°C, more preferably 18 to 30°C.

A further advantage of the present method of the invention is that the method does not require a deoxygenation step since the formulations obtained are stable against oxygenation and can be stored at air. Therefore, a preferred embodiment of the method according to the present invention does not comprise a deoxygenation step.

A further embodiment of the present invention is a container containing a composition of the present invention. The container can be made of an organic polymer.

Since the compositions of the present inventions are not sensitive with respect to oxidation caused by oxygen from the atmosphere container made of organic polymers which may be permeable for oxygen can be used. Containers, such as vials made of organic polymers are advantageous since they do not break and the handling is much easier.

Preferably, the organic polymer is semipermeable for oxygen, preferably selected from polyethylene or polypropylene.

According to a further embodiment the container comprising the composition according to the present invention is a container made of an impermeable material for oxygen, preferably a glass material.

A further embodiment of the present invention is the use of hydroxyethyl starch, preferably a hydroxyethyl starch as defined above, for the increase of the rate of dissolution of acetaminophen in an aqueous solution. Preferably, the hydroxyethyl starch is used for dissolving acetaminophen, preferably at temperatures ranging from 5 to 50°C, more preferably at 15 to 40°C and especially at 18 to 30°C.

### Examples

### I. Compositions F1 to F4

4 different compositions of the invention (Fl to F4) were prepared and the effect of hydroxyethylstarch (HES) relative to the rate of dissolution of acetaminophen powder (identical in each of F1 to F4) was determined. The rate of dissolution has been determined at 22°C.

The amounts of components referred to in Table 1 are in weight percent (wt.-%).

**Table 1: Composition F1 to F4 of the invention**

| Components | F1 | F2 | F3 | F4 |
|---|---|---|---|---|
| Acetaminophen | 1.00 | 1.00 | 1.00 | 1.00 |
| Mannitol | 3.10 | 3.10 | 3.10 | 3.10 |
| HES¹⁾ | 0.10 | 0.2 | 0.5 | 1.0 |
| Sodium acetate trihydrate | 0.30 | 0.30 | 0.30 | 0.30 |
| Sodium citrate dihydrate | 0.30 | 0.30 | 0.30 | 0.30 |
| Acetic acid in an amount to adjust at pH 5.5 | pH at 5.5 | pH at 5.5 | pH at 5.5 | pH at 5.5 |
| Water for injection | ad 100 | ad 100 | ad 100 | ad 100 |
| Solubility at 22°C | + | + | ++ | +++ |

| | | | | |
|---|---|---|---|---|
| ¹⁾hydroxyethylstarch with an average molecular weight of 70000 and a molar substitution (MS) of 0.4 | | | | |

The rate of dissolution of the compositions has been determined visually taking into account the time needed to completely dissolve the acetaminophen. The observations are classified in the following order where "+++" denotes highest rate of dissolution which means shortest time to completely dissolve acetaminophen and "+" denotes lowest rate of dissolution which means longest time needed to completely dissolve acetaminophen.

### II. Compositions C1 to C10 and E1

11 different compositions were prepared (C1 to C10 correspond to comparative examples and E1 is an example according to the invention) in order to determine the effect of the selected components and the stability of acetaminophen in an aqueous solution.

The amounts referred to in Table 2 are in weight percent.

**Table 2: Compositions C1 to C10 and E1**

| Active Ingredients | | C1 | C2 | C3 | C4 | C5 | C6 | C7 | E1 | C8 | C9 | C10 |
|---|---|---|---|---|---|---|---|---|---|---|---|---|
| Paracetamol | | 1,000 | 1,000 | 1,000 | 1,000 | 1,000 | 1,000 | 1,000 | 1,000 | 1,000 | 1,000 | 1,000 |
| HES ¹⁾ | | | | | | | | | 0,100 | | | |
| Dextrane ²⁾ | | | | | | | | | | | 0,100 | |
| Solutol ³⁾ | | | | | | 0,100 | 0,100 | 0,100 | | | | |
| Kollindon ⁴⁾ | | | 0,100 | 0,100 | 0,100 | | | | | | | |
| Buffering Agent | | | | | | | | | | | | |
| Disodium hydrogene phosphate 2-H2O | | 0,120 | 0,120 | 0,120 | 0,120 | 0,120 | 0,120 | 0,120 | 0,120 | 0,120 | 0,120 | 0,120 |
| Osmolality Agent | | | | | | | | | | | | |
| Glucose | | | | 4,000 | | | 4,000 | | 4,000 | | 4,000 | |
| Mannitol | | 4,000 | 3,500 | | | 3,500 | | | | | | |
| Sodium Glycerophosphate pentahydrate | | | | | 3,000 | | | 3,000 | | 3,000 | | |
| Gluconolactone | | | | | | | | | | | | 4,000 |
| pH adjustment | | | | | | | | | | | | |
| Phosphoric acid in an amount to adjust pH 5.5 | | | | | | | | | | | | |
| Water | | ad 100 | ad 100 | ad 100 | ad 100 | ad 100 | ad 100 | ad 100 | ad 100 | ad 100 | ad 100 | ad 100 |
| Stability after 15 days at | 40°C | colourless | colourless | colourless | pink | colourless | colourless | pink | colourless | pink | colourless | yellow |
| | 55°C | virtually colourless | virtually colourless | slightly yellow | pinkish brown | virtually colourless | slightly yellow | pinkish brown | colourless | pinkish brown | slightly yellow | yellowish brown |
| | 70°C | yellow brown | mellow | yellowish brown | brown | yellow | yellowish brown | brown | yellow | brown | yellowish | brown |

| | | | | | | | | | | | | |
|---|---|---|---|---|---|---|---|---|---|---|---|---|
| 1) hydroxyethylstarch with an average molecular weight of 70000 and a molar substitution (MS) of 0.4 2) Dextrane with an average molecular weight of 70000 3) Solutol^{®} HS-15 ex BASF (polyethylene glycol-15-hydroxystearate) 4) Kollindon^{®}12 PF ex BASF (polyvinyl pyrrolidone) | | | | | | | | | | | | |

The stability of the composition has been determined by monitoring the degree of coloration (which reflects the product stability) of each formulation after 15 days storage at 40, 55 and 70 °C in a 50 ml glass vial container (with rubber stopper and metallic capsule). These samples were sterilized in an autoclave at 121°C/15 minutes prior to the test procedure.

Stability of the compositions has been determined visually and classified in the following order where "colourless" denotes highest stability and "black, visible parts" denotes lowest stability:
colourless > virtually colourless > slightly colourless > slightly brown > slightly yellow > brownish >yellowish > pink>pinkish brown >yellow > yellowish brown > brown > intense brown > intense yellowish brown >intense yellow > intense brown, visible particles > black, visible particles

From the results provided in Table 2, it is clear that hydroxyethylstarch (HES) plays an important role in the stabilization of Paracetamol in an aqueous solution allowing the dissolution of paracetamol in aqueous solution at room temperature and establishing positive interaction with the paracetamol in aqueous solution.

### III. Compositions E2 to E6 and C11 to C15

Compositions E2 to E6 according to the invention are compared with compositions C11 to C15 not according to the invention (see Table 3).

The amounts referred to in Table 3 are in percent by weight.

**Table 3: Examples E2 to E6 and C11 to C15**

| Components | | E2 | E3 | E4 | E5 | C11 | C12 | E6 | C13 | C14 | C15 |
|---|---|---|---|---|---|---|---|---|---|---|---|
| Active Ingredients | | | | | | | | | | | |
| Paracetamol | | 1,000 | 1,000 | 1,000 | 1,000 | 1,000 | 1,000 | 1,000 | 1,000 | 1,000 | 1,000 |
| HES ¹⁾ | | 0,100 | 1,000 | 0,100 | 1,000 | | | 1,000 | | | |
| Solutol ²⁾ | | | | | | 0,100 | 0,100 | | | | |
| L-Cysteine HCl | | | | | | | | | 0,025 | | |
| Buffering and osmolating agent | | | | | | | | | | | |
| Disodium hydrogen phosphate 12-H₂O | | 0,120 | 0,120 | 0,120 | 0,120 | 0,120 | 0,120 | 0,120 | 0,013 | | |
| Sodium Acetate 3H₂O | | | | | | | | | | 0,300 | 0,300 |
| Sodium Citrate 2H₂O | | | | | | | | | | 0,300 | 0,300 |
| Glucose | | | | 4,000 | 4,000 | 4,000 | | | | 3,300 | 3,300 |
| Mannitol | | 3,600 | 3,600 | | | | 3,600 | | 3,500 | | |
| Sodium Chloride | | | | | | | | 0,800 | | | |
| HCl/NaOH in an amount to adjust to pH 5.5 | | | | | | | | | | | |
| Sodium Formaldehyde sulfosilate | | | | | | | | | | | 0,020 |
| Water | | ad 100 | ad 100 | ad 100 | ad 100 | ad 100 | ad 100 | ad 100 | ad 100 | ad 100 | ad 100 |
| Stability after 15 days at | 55°C | virtually colourless | virtually slightly colourless | brown | slightly brown | slightly brown | slightly brown | pink | brown | silghtly brown | slightly brown |
| | 70°C | brown | brown | Intense brown | brown | brown | intense brown | brown | intense brown, visible particles | intense | intense yellowish brown |

| | | | | | | | | | | | |
|---|---|---|---|---|---|---|---|---|---|---|---|
| 1) hydroxyethylstarch with an average molecular weight of 70000 and a molar substitution (MS) of 0.4 2) Solutol^{®} HS-15 ex BASF (polyethylene glycol-15-hydroxystearate) | | | | | | | | | | | |

The stability of the composition has been determined by monitoring the degree of coloration (which reflects the product stability) of each formulation after 15 days storage at 55 and 70 °C in a 50 ml glass vial container (with rubber stopper and metallic capsule). These samples were sterilized in an autoclave at 121°C/15 minutes prior to the test procedure.

Stability of the compositions has been determined visually and classified in the following order where "colourless" denotes highest stability and "black, visible parts" denotes lowest stability:
colourless > virtually colourless > slightly colourless > slightly brown > slightly yellow > brownish >yellowish > pink>pinkish brown >yellow > yellowish brown > brown > intense brown > intense yellowish brown >intense yellow > intense brown, visible particles > black, visible particles

The composition according to the invention (E2 to E5) are significantly more stable in terms of hydrolysis of the paracetamol as well as the oxidative degradation of the paracetamol.

Further, mannitol appears to be the most suitable non ionic osmolality agent for giving a suitable isoosmolality to the formulation and at the same time for controlling the ionic strength of the composition.

### IV. Compositions E7 to E10 and C16 to C22

Comparison of the compositions of the invention (E7 to E10) and compositions not according to the invention (C16 to C22) in plastic semi-permeable container (polyethylen).

Compositions E7 to E10 and C16 to C22 were stored in semipermeable container (polyethylene) which were sealed under an air atmosphere (21% oxygen).

The amounts of the components referred to in Table 4 are in percent by weight.

**Table 4: Compositions E7 to E10 and C16 to C22**

| Active Ingredients | | E10 | E7 | E8 | E9 | C16 | C17 | C18 | C19 | C20 | C21 | C22 |
|---|---|---|---|---|---|---|---|---|---|---|---|---|
| Paracetamol | | 1,000 | 1,000 | 1,000 | 1,000 | 1,000 | 1,000 | 1,000 | 1,000 | 1,000 | 1,000 | 1,000 |
| HES¹⁾ | | 0,100 | 1,000 | 0,100 | 1,000 | | | | | | | |
| Solutol²⁾ | | | | | | 0,100 | | 0,100 | | | | |
| Kollindon³⁾ | | | | | | | 0,100 | | 0,100 | | | |
| L-Cysteln HCl | | | | | | | | | | 0,025 | | |
| Buffering and osmolating excipients | | | | | | | | | | | | |
| Disodium hydrogene phosphate 12H₂O | | 0,120 | 0,120 | | | 0,120 | 0,120 | | | 0,013 | | |
| Sodium dihydrogen phosphate 2H₂O | | | | | | | | | | | | |
| Sodium Acetate 3H2O | | | | 0,300 | 0,300 | | | 0,300 | 0,300 | | 0,300 | 0,300 |
| Sodium Citrate 2H2O | | | | 0,300 | 0,300 | | | 0,300 | 0,300 | | 0,300 | 0,300 |
| Glacial acetic acid amount to adjust in an at pH 5.5 | | | | X ml | X ml | | | X ml | X ml | | X ml | X ml |
| Glucose | | | | | | | | | | | 3,300 | 3,300 |
| Mannitol | | 3,600 | 3,600 | 3,600 | 3,600 | 3,600 | 3,600 | 3,300 | 3,300 | 3,500 | | |
| HCl/NaOH in an adjust at pH 5.5 amount to | | | | | | | | | | X ml | | |
| Sodium Formaldehyde sulfosilate | | | | | | | | | | | | 0,020 |
| Phosphoric acid | | X ml | X ml | | | X ml | X ml | | | | | |
| Water | | ad 100 | ad 100 | ad 100 | ad 100 | ad 100 | ad 100 | ad 100 | ad 100 | ad 100 | ad 100 | ad 100 |
| Stability after 15 days at | 55°C | slightly brown | slightly brown | slightly brown | slightly brown | slightly brown | slightly brown | yellowish | yellowish | brown | yellowish | slightly yellow |
| | 70°C | brown | brown | brownish | brownish | intense brown | intense brown | intense yellow | intense yellow | black, visible particles | Intense brow | intense brown |

| | | | | | | | | | | | | |
|---|---|---|---|---|---|---|---|---|---|---|---|---|
| 1) hydroxyethylstarch with an average molecular weight of 70000 and a molar substitution (MS) of 0.4 2) Solutol^{®} HS-15 ex BASF (polyethylene glycol-15-hydroxystearate) 3) Kollindon^{®}12 PF ex BASF (polyvinyl pyrrolidone) | | | | | | | | | | | | |

The stability of the composition has been determined by monitoring the degree of coloration (which reflects the product stability) of each formulation after 15 days storage at 55 and 70 °C in 100 ml sealed semipermeable polyethylene containers which were sterilized at the beginning of the test procedure by an autoclave at 112°C/70 minutes.

Stability of the compositions has been determined visually and classified in the following order where "colourless" denotes highest stability and "black, visible parts" denotes lowest stability:
colourless > virtually colourless > slightly colourless > slightly brown > slightly yellow > brownish >yellowish > pink>pinkish brown >yellow > yellowish brown > brown > intense brown > intense yellowish brown >intense yellow > intense brown, visible particles > black, visible particles

The Comparative Examples show that if prepared without a deoxygenation step (N₂ bubbling) are even less stable in a semi-permeable plastic container (an intensive brown coloration appears after sterilization in an autoclave). The most stable formulations are the composition with mannitol, HES and acetate/citrate as a buffering agent (E8 and E9).

Further, it is demonstrated that the compositions of the invention are stable without deoxygenation (N₂ bubbling).

### V. Demonstration of the independence on the oxygen content

In order to demonstrate that the composition of the invention is not dependent on the oxygen content, composition E11 referred to in Table 5 has been prepared under the following conditions:

| | |
|---|---|
| G1: | During the dissolution of the acetaminophen with the other components of composition E11 no deoxygenation step is applied. Further, during the filling of the composition in glass vials no deoxygenation step is applied. The oxygen (O₂) content in the liquid composition is 8.7 pm and the O₂ content in the air in the head space of the vial is 21%. |
| | |
| G2: | During the dissolution of the acetaminophen with the other components of composition E11 no deoxygenation step is applied. Further, during the filling of the composition in glass vials a nitrogen stream is used to reduce the oxygen content in the head space of the glass vial. The O₂ content in the liquid composition is 8.7 ppm and the O₂ content in the air in the head space of the vial is 3%. |
| | |
| G3: | During the dissolution of the acetaminophen with the other components of composition E11 nitrogen (N₂) is bubbled through the aqueous mixture. However, during the filling glass vials no deoxygenation step is applied. The oxygen (O₂) content in the liquid composition is 0.1 ppm and the O₂ content in the head space of the vial is 21%. |
| | |
| G4: | During the dissolution as well as during the filling the composition is deoxygenated with N₂. The O₂ content of the liquid composition is 0.1 ppm and the O₂ content in the air in the head space of the glass vial is 3%. |

Table 6 shows the results in terms of stability for composition E11 prepared under the conditions G1 to G4.

**Table 5: Composition E11**

| Liquid injectable composition according to the invention | |
|---|---|
| Paracetamol | 1.00 |
| Mannitol | 3.10 |
| HES 70000¹⁾ | 1.00 |
| Sodium Acetate Trihydrate | 0.30 |
| Sodium Citrate Dihydrate | 0.30 |
| Acetic Acid 50% (v/v) | to adjust pH at 5.5 |
| Water for injections | ad 100 |

| | |
|---|---|
| 1) hydroxyethylstarch with an average molecular weight of 70000 and a molar substitution (MS) of 0.4 | |

**Table 6: Stability of E11 under conditions G1 to G4**

| | | G1 | G2 | G3 | G4 |
|---|---|---|---|---|---|
| Stability after 22 days at | 25°C | colourless | colourless | colourless | colourless |
| | 30°C | colourless | colourless | colourless | colourless |
| | 40°C | virtually colourless | virtually colourless | virtually colourless | virtually colourless |
| | 55°C | slightly brown | slightly brown | slightly brown | slightly brown |
| | 70°C | brownish | brownish | brownish | brownish |

The stability of the composition has been determined by monitoring the degree of coloration (which reflects the product stability) of each formulation after 22 days storage at (25, 30, 40, 55 and 70 °C in 100 ml sealed semipermeable polyethylene containers which were sterilized prior to the test procedure by an autoclave at 112°C/70 minutes.

Stability of the compositions has been determined visually and classified in the following order where "colourless" denotes highest stability and "black, visible particles" denotes lowest stability:
colourless > virtually colourless > slightly colourless > slightly brown > slightly yellow > brownish >yellowish > pink>pinkish brown >yellow > yellowish brown > brown > intense brown > intense yellowish brown >intense yellow > intense brown, visible particles > black, visible particle

The results presented in Table 6 show that the compositions according to the invention are stable independent of the oxygen present in the environment, i.e. the oxygen content in the composition or in the atmosphere surrounding the composition.

## Claims

1. Liquid injectable composition comprising
a) acetaminophen,
b) hydroxyethyl starch and
c) at least one osmolality agent.

2. Composition according to claim 1 further comprising a buffering agent, preferably selected from the group consisting of a buffer based on acetate, citrate and phosphate as well as mixtures thereof.

3. Composition according to claim 1 or 2 wherein the composition has a pH value ranging from 4 to 8, preferably 4.5 to 6.5, more preferably from 5.0 to 6.0.

4. Composition according to at least one of the preceding claims wherein the composition is aqueous.

5. Composition according to at least one of the preceding claims wherein the osmolality agent is an aliphatic polyhydroxy alkanol having 2 to 10 carbon atoms, preferably selected from the group consisting of mannitol, fructose, glucose, gluconolactone, gluconate and mixtures thereof.

6. Composition according to at least one of the preceding claims wherein the osmolality agent is mannitol.

7. Pharmaceutical composition comprising at least a liquid injectable composition as defined in at least one of the preceding claims.

8. Pharmaceutical composition according to claim 7 for use in the prophylaxis and treatment of pain and/or fever.

9. Method for the manufacturing of a composition as defined in at least one at the claims 1 to 8 comprising the step:
dissolving acetaminophen in a solvent in the presence of hydroxyethyl starch

10. Method according to claim 9 wherein the solvent is an aqueous solvent, preferably water.

11. Method according to claim 9 or 10 wherein the acetaminophen is dissolved in the presence of hydroxyethyl starch at a temperature ranging from 5 to 50°C, preferably 15 to 40°C, more preferably 18 to 30°C.

12. Method according to at least one of claims 9 to 11 not comprising a deoxygenation step.

13. Container containing a composition according to claims 1 to 8.

14. Container according to claim 13 made of an organic polymer or glass.

15. Use of hydroxyethyl starch for the increase of the rate of dissolution of acetaminophen in an aqueous solution.

## Patentansprüche

1. Flüssige injizierbare Zusammensetzung, umfassend:
a) Acetaminophen;
b) Hydroxyethylstärke; und
c) wenigstens ein Osmolalitätsmittel.

2. Zusammensetzung gemäß Anspruch 1, weiterhin umfassend ein Puffermittel, das vorzugsweise aus der Gruppe ausgewählt ist, die aus einem Puffer auf Acetat-, Citrat- und Phosphatbasis sowie Gemischen davon besteht.

3. Zusammensetzung gemäß Anspruch 1 oder 2, wobei die Zusammensetzung einen pH-Wert im Bereich von 4 bis 8, vorzugsweise 4,5 bis 6,5, besonders bevorzugt 5,0 bis 6,0, aufweist.

4. Zusammensetzung gemäß wenigstens einem der vorstehenden Ansprüche, wobei die Zusammensetzung wässrig ist.

5. Zusammensetzung gemäß wenigstens einem der vorstehenden Ansprüche, wobei das Osmolalitätsmittel ein aliphatischer Polyhydroxyalkohol mit 2 bis 10 Kohlenstoffatomen ist, der vorzugsweise aus der Gruppe ausgewählt ist, die aus Mannit, Fructose, Glucose, Gluconolacton, Gluconat und Gemischen davon besteht.

6. Zusammensetzung gemäß wenigstens einem der vorstehenden Ansprüche, wobei es sich bei dem Osmolalitätsmittel um Mannit handelt.

7. Pharmazeutische Zusammensetzung, die wenigstens eine flüssige injizierbare Zusammensetzung gemäß wenigstens einem der vorstehenden Ansprüche umfasst.

8. Pharmazeutische Zusammensetzung gemäß Anspruch 7 zur Verwendung bei der Prophylaxe und Behandlung von Schmerzen und/oder Fieber.

9. Verfahren zur Herstellung einer Zusammensetzung gemäß wenigstens einem der Ansprüche 1 bis 8, umfassend den Schritt:
Auflösen von Acetaminophen in einem Lösungsmittel in Gegenwart von Hydroxyethylstärke.

10. Verfahren gemäß Anspruch 9, wobei das Lösungsmittel ein wässriges Lösungsmittel, vorzugsweise Wasser, ist.

11. Verfahren gemäß Anspruch 9 oder 10, wobei das Acetaminophen in Gegenwart von Hydroxyethylstärke bei einer Temperatur im Bereich von 5 bis 50 °C, vorzugsweise 15 bis 40 °C, besonders bevorzugt 18 bis 30 °C, aufgelöst wird.

12. Verfahren gemäß wenigstens einem der Ansprüche 9 bis 11, das keinen Deoxygenierungsschritt umfasst.

13. Behälter, der eine Zusammensetzung gemäß den Ansprüchen 1 bis 8 enthält.

14. Behälter gemäß Anspruch 13, der aus einem organischen Polymer oder Glas besteht.

15. Verwendung von Hydroxyethylstärke zur Erhöhung der Auflösungsgeschwindigkeit von Acetaminophen in einer wässrigen Lösung.

## Revendications

1. Composition liquide injectable, comprenant
a) de l'acétaminophène,
b) de l'hydroxyéthylamidon, et
c) au moins une substance osmotiquement active.

2. Composition selon la revendication 1, comprenant en outre un agent de tamponnement, de préférence choisi dans le groupe consistant en un tampon à base d'acétate, de citrate et de phosphate, ainsi que des mélanges de ceux-ci.

3. Composition selon la revendication 1 ou 2, dans laquelle la composition a une valeur pH comprise entre 4 et 8, de préférence entre 4,5 et 6,5, de préférence encore entre 5,0 et 6,0.

4. Composition selon au moins une des revendications précédentes, dans laquelle la composition est aqueuse.

5. Composition selon au moins une des revendications précédentes, dans laquelle la substance osmotiquement active est un polyalcool aliphatique ayant 2 à 10 atomes de carbone, de préférence choisi dans le groupe consistant en mannitol, fructose, glucose, gluconolactone, gluconate, et des mélanges de ceux-ci.

6. Composition selon au moins une des revendications précédentes, dans laquelle la substance osmotiquement active est le mannitol.

7. Composition pharmaceutique comprenant au moins une composition liquide injectable telle que définie dans au moins une des revendications précédentes.

8. Composition pharmaceutique selon la revendication 7 destinée à être utilisée pour la prophylaxie et le traitement de la douleur et/ou de la fièvre.

9. Procédé pour fabriquer une composition telle que définie dans au moins une des revendications 1 à 8, comprenant l'étape consistant à :
dissoudre de l'acétaminophène dans un solvant en la présence d'hydroxy-éthylamidon.

10. Procédé selon la revendication 9, dans lequel le solvant est un solvant aqueux, de préférence de l'eau.

11. Procédé selon la revendication 9 ou 10, dans lequel l'acétaminophène est dissous en la présence d'hydroxyéthylamidon à une température comprise entre 5 et 50 °C, de préférence entre 15 et 40 °C, de préférence encore entre 18 et 30 °C.

12. Procédé selon au moins une des revendications 9 à 11 ne comprenant pas d'étape de désoxygénation.

13. Récipient contenant la composition selon les revendications 1 à 8.

14. Récipient selon la revendication 13 étant réalisé en polymère organique ou en verre.

15. Utilisation d'hydroxyéthylamidon pour augmenter la vitesse de dissolution de l'acétaminophène dans une solution aqueuse.
